# EUROPEAN PATENT SPECIFICATION

(11) **EP 2 964 276 B1**
(45) Date of publication and mention of the grant of the patent: **07.02.2018**
(21) Application number: 14708848.8
(22) Date of filing: 05.03.2014
(51) Int. Cl.: A61L 26/00, A61L 24/00

(54) **MEDICAL PRODUCT IN THE FORM OF A FIBROUS FLOCK MATERIAL; SUSPENSION THEREOF**
MEDIZINISCHES PRODUKT IN FORM VON FASERFÖRMIGEM FLOCKENMATERIAL, SUSPENSION DAFÜR
PRODUITS MÉDICAUX SOUS FORME DE FLOCONS FIBREUX ; SUSPENSION ASSOCIÉE

(30) Priority: 08.03.2013 DE 102013203997
(43) Date of publication of application: 13.01.2016
(73) Proprietor: Aesculap AG, 78532 Tuttlingen (DE)
(72) Inventor: MERCKLE, Christof, 68199 Mannheim (DE); ODERMATT, Erich, 8200 Schaffhausen (CH)
(74) Representative: Patentanwälte Ruff, Wilhelm, Beier, Dauster & Partner mbB
(86) International application number: PCT/EP2014/054213
(87) International publication number: WO 2014/135562

(56) References cited:
- US-A1- 2007 031 474
- US-A1- 2010 042 034
- US-B1- 6 203 563

## Description

### Field of application and prior art

The invention relates to a medical product and to the fields of application thereof in the area of medicine.

Medical products such as, for example, spongy or foamy wound coverings are described in EP 2 233 157 A1.

An occasional problem in the case of the medical products known from the prior art is their application into poorly accessible body defect zones.

Moreover, there is a certain risk that - depending on the material used - contracting of the material and thus an inhomogeneous distribution of the material in a body defect zone can occur, endangering the effect which is intended from a medical point of view.

A further problem in the case of products known from the prior art is that they do not always optimally promote wound healing.

US 2007/0031474 A1 discloses implantable globin preparations which may be in the form of injectable powders, pastes, gels, suspensions and solutions or implantable solid materials.

US 6,203,563 B1 discloses a multicomponent healing device for placing and compacting a mass of polymer fiber flock into a wound.

US 2010/0042034 A1 relates to a wound care article containing fibers which are arranged in the form of planar fabric bands.

### Problem and solution

Against this background, the problem addressed by the present invention is therefore that of providing a technical solution which adequately addresses the disadvantages discussed above.

This problem is solved by a medical product having the features of independent claim 1 and by a medical kit having the features of claim 11. Preferred embodiments of the medical product are defined in claims 2 to 10. The wording of all the claims is hereby made content of the description by express reference. Apart from that, the problem addressed by the invention is solved by further subjects of the invention which are dealt with exclusively in the description.
In a first aspect, the invention provides a medical product in the form of a fibrous flock material. Alternatively, the medical product can be present in the form of a suspension which contains the fibrous flock material.
The invention is based on the surprising finding that fibrous flock material as such is usable as a medical product or for producing medical suspensions.
The medical product according to the invention has the following advantages:
» Owing to the fibrous design of the flock material, the medical product has an enlarged surface area, and this is an advantage especially in the case of wound coverings, in the case of stopping bleeding and/or of wound healing. Wound healing can be additionally improved by additization with wound healing-promoting substances.
» After application of the medical product to or into a body defect zone, the fibrous flock material can, with particular advantage, form a kind of "three-dimensional structure" or "matrix" having cavities or pores (between the applied flock particles and/or fibres), favouring the ingrowth of cells, extracellular matrix or tissue and thus the wound healing process.
» A further advantage associated with the enlarged surface area of the flock material is that generally relatively low amounts of the medical product are sufficient in order to achieve the effect desired from a medical point of view.
» A further advantage of the medical product arising from the fibrous design of the flock material is that the product can be distributed more uniformly or more homogeneously on or in a body defect zone, ensuring that sufficient material for achieving the intended medical effect is available on or in the body defect zone.
   Any visible inhomogeneities in the distribution of the medical product can be easily compensated for by "re-application" of the product before or during a treatment step.
» A further advantage is that, owing to the fibrous design of the flock material, the medical product allows complete coverage of a body defect zone to be treated, for example a wound, without complicated adaptations of the product to the proportions of the body defect zone to be treated being necessary before or during a medical procedure, more particularly a surgical procedure.
» Another advantage is that, owing to the fibrous design of the flock material, the medical product can be applied in a targeted manner to or into a body defect zone.
» Owing to the fibrous design of the flock material and the associated surface area enlargement, it is additionally possible to more easily and, in particular, more rapidly apply the medical product to or into a body defect zone. Depending on the form of the product, the latter can be applied as, for example, a dispersion or suspension by means of spraying (or optionally by means of spreading) or a paste by means of brushing to or into the body defect zone. This likewise contributes to facilitating the desired treatment.
» As a result, the medical product can also be applied without relatively great difficulties to or into poorly accessible body defect zones.
» A further advantage is that, owing to the fibrous design of the flock material, the medical product can be applied to or into a body defect zone in reproducible and, in particular, definable amounts, making it possible to carry out a medical procedure, more particularly a surgical procedure, under similarly reproducible and, in particular, definable conditions.
» Through the specific selection of materials and/or additives for the flock material, it is additionally possible to specifically influence or adjust the properties thereof such as, for example, absorbency, swellability, adhesiveness and resorbability. This allows indication-specific adaptation of the product according to the invention.
» Lastly, one advantage is that the flock material and thus the product according to the invention can be produced in a cost-effective manner. For instance, the flock material can, for example, be extruded and transferred to a fibrous form.

In the context of the present invention, the term "fibrous flock material" or "flock fibres" is to be understood to mean fibres having a defined fibre length (preferred fibre lengths will be mentioned below). The fibrous flock material is usually produced by trimming to length, more particularly cutting, or milling of single fibres (for example extrusion fibres), more particularly so-called continuous fibres.

The medical product is preferably a wound covering agent, more particularly a surgical wound covering agent.

The medical product is present especially in the form of a spray bandage.

The medical product is preferably suitable for use in the treatment of surgical wounds and/or for the treatment of external wounds such as, for example, burn wounds and/or chronic wounds and/or for the treatment of internal wounds such as, for example, parenchymatous wounds.

A further possible area of application concerns the use of the medical product for use in the treatment of ulcers.

In a further embodiment, the medical product is a haemostatic (styptic), i.e. an agent for stopping bleeding.

The medical product can be intended especially for use in the treatment of diffuse bleeding and/or heavy bleeding such as, for example, parenchymatous bleeding.

In a further embodiment, the medical product is a filling material, especially for filling in or augmenting soft tissue defects such as, for example, cartilage defects and/or hard tissue defects such as, for example, bone defects.

In a further embodiment, the medical product is an occlusion agent.

In a further embodiment, the medical product is intended for closing, occluding or for sealing dura mater defects.

In a further embodiment, the medical product is intended for sealing or occluding air and/or liquid leaks in the human and/or animal body.

In a further embodiment, the medical product is intended for sealing or occluding puncture channel bleeding.

In a further embodiment, the medical product is intended for use in negative-pressure therapy or negative-pressure sealing.

In a preferred embodiment, the fibrous flock material is flock fibres, preferably monofilament flock fibres.

The fibrous flock material is present in the form of single flock fibres. In other words, the fibrous flock material is not connected, especially not via an adhesive layer, to a substrate, more particularly an implant body and/or a supporting element, or assembled to form a planar structure such as, for example, a non-woven fabric, non-woven scrim, loop-drawingly knitted fabric, loop-formingly knitted fabric or the like.

The fibrous flock material can, in principle, have a fibre length of from 40 µm to 15 mm, more particularly from 50 µm to 10 mm, preferably from 100 um to 5 mm. The fibrous flock material can, in particular, have a fibre length of from 10 µm to 5 mm, more particularly from 40 µm to 4 mm, preferably from 50 µm to 3 mm, more preferably from 100 µm to 2 mm. The fibrous flock material can, with particular preference, have a fibre length of from 100 µm to 3 mm, preferably from 200 µm to 2 mm.

Furthermore, the fibrous flock material can have a fibre thickness or fibre diameter of from 20 µm to 900 µm, more particularly from 70 µm to 600 µm, preferably from 100 µm to 400 µm.

In addition, the fibrous flock material can have a linear density of from 0.01 dtex to 1000 dtex, more particularly from 0.1 dtex to 500 dtex, preferably from 0.3 dtex to 200 dtex. The fibrous flock material can, in particular, have a linear density of from 0.01 dtex to 100 dtex, preferably from 0.1 dtex to 80 dtex, more preferably from 0.3 dtex to 40 dtex. The dimension "dtex" (de-citex) means a linear density of 1 g per 10 000 m length of the fibrous flock material.

In a further embodiment, the fibrous flock material can be textured or non-textured.

Furthermore, the fibrous flock material can have a circular cross section or a non-circular cross section, more particularly an oval, ellipsoidal, polygonal, for example triangular, rectangular, square, rhomboidal, pentagonal, hexagonal and/or star-shaped, cross section.

In a further embodiment, the flock material comprises a mucoadhesive material or is formed from such a material. More particularly, the flock material can comprise a material or be formed from a material which is capable of binding to biological tissue, more particularly human and/or animal tissue, via ionic interactions and/or the formation of hydrogen bonds. In a further embodiment, suitable materials comprise functional groups preferably selected from the group comprising carboxylate groups, phosphate groups, sulphate groups, sulphonate groups, amino groups and combinations thereof. The embodiments described in this paragraph are especially advantageous with regard to wound-covering and/or haemostatic uses of the product according to the invention.

To increase the dimensional stability of the product according to the invention, it can additionally be provided that the flock material comprises a crosslinked material or is formed from such a material. A dimensionally stable product is especially advantageous with regard to use as filling material. In this case, the material can be physically and/or chemically crosslinked. It is possible to achieve physical crosslinking of the material by means of, for example, irradiation techniques suitable for this purpose. Corresponding techniques are familiar to a person skilled in the art, and so further explanations will not be given here. By contrast, chemical crosslinking of the material is generally based on the use of a suitable crosslinker. For example, the flock material can be crosslinked with a chemical crosslinker selected from the group comprising aldehydes such as, for example, formaldehyde, dialdehydes such as, for example, glutaraldehyde, polyaldehydes such as, for example, dextran aldehyde, carbodiimides, diisocyanates such as, for example, hexamethylene diisocyanate, salts thereof and mixtures thereof.

The flock material comprises a resorbable material or is formed from such a material. In this embodiment, the surface area enlargement associated with the fibrous design of the flock material promotes rapid resorption of the medical product.

In addition, the flock material can comprise a non-resorbable material. From a dimensional stability point of view and from a load-bearing point of view, a non-resorbable flock material can be especially advantageous when using the product according to the invention as filling material.

In a further embodiment, the material described in the preceding embodiments for the flock material can be of synthetic, biological and/or recombinant origin.

Furthermore, the material for the flock material can be of natural or native origin. The material can, in particular, be of xenogeneic origin, preferably porcine, bovine and/or equine origin. Alternatively, the material for the flock material can also be of human origin.

Preferably, the material for the flock material is a polymer, selected in particular from the group comprising synthetic polymer, recombinant polymer, naturally occurring polymer or biopolymer and mixtures, more particularly blends, thereof. More particularly, the polymer can be a copolymer. In the context of the present invention, the expression "copolymer" is to be understood to mean a polymer composed of at least two different monomer units. Thus, the expression "copolymer" encompasses not only copolymers in the narrower sense, i.e. so-called bipolymers (polymers composed of two different monomer units), but also terpolymers, tetrapolymers, etc. The copolymer can additionally be selected from the group comprising random copolymer, alternating copolymer, block copolymer or segmented copolymer, graft copolymer and mixtures, more particularly blends, thereof.

Preferably, the flock material comprises a resorbable material or is formed from such a material which is selected from the group comprising polyhydroxyalkanoates, proteins, extracellular proteins, serum proteins, polysaccharides, mucopolysaccharides, carboxyl group-bearing polysaccharides, amino group-bearing polysaccharides, aldehyde group-bearing polysaccharides, copolymers thereof, salts thereof, stereoisomers, more particularly diastereomers, thereof and mixtures, more particularly blends, thereof.

More particularly, the flock material can comprise a resorbable material or be formed from such a material which is selected from the group comprising polyglycolide or polyglycolic acid, polylactide or polylactic acid, polydioxanone, poly-3-hydroxybutyrate or poly-3-hydroxybutyric acid, poly-4-hydroxybutyrate or poly-4-hydroxybutyric acid, polytrimethylene carbonate, poly-ε-caprolactone, polyvinyl alcohol, cotton, cellulose, cellulose derivatives such as, for example, alkylcelluloses, methylcellulose, hydroxyalkylcelluloses, hydroxymethylcellulose, hydroxyethylcellulose, hydroxypropylcellulose, carboxyalkylcelluloses, carboxymethylcellulose, starch, amylose, amylopectin, dextran, dextrin, chitin, chitosan, hyaluronic acid, dextran sulphate, heparin, heparan sulphate, chondroitin sulphate, dermatan sulphate, collagen, gelatin, elastin, reticulin, fibronectin, laminin, fibrin, fibrinogen, albumin, copolymers thereof, salts thereof, stereoisomers, more particularly diastereomers, thereof and mixtures, more particularly blends, thereof.

In addition, the flock material can comprise a non-resorbable material which is selected from the group comprising polyolefins, polyamides, polyesters, polyurethanes, more particularly thermoplastic polyurethanes, copolymers thereof and mixtures, more particularly blends, thereof.

More particularly, the flock material can comprise a non-resorbable material which is selected from the group comprising polyethylene, low-density polyethylene, high-density polyethylene, high-molecular-weight polyethylene, ultra-high-molecular-weight polyethylene, polypropylene, polyethylene terephthalate, polypropylene terephthalate, polybutylene terephthalate, polyacrylonitrile, nylon-6, nylon-6,6, nylon-6,12, nylon-12, silk, more particularly rayon or spider silk, polytetrafluoroethylene, more particularly expanded polytetrafluoroethylene (ePTFE), polyvinylidene difluoride, polytetrafluoropropylene, polyhexafluoropropylene, copolymers thereof and mixtures, more particularly blends, thereof.

In a further embodiment, the flock material can comprise a material having a melting point below 100 °C, in particular having a melting point from 70 °C to 100 °C, or can be formed of such a material.

In advantageous embodiments, the flock material can be additized, i.e. comprise one or optionally more additives.

To achieve detectability, especially by means of imaging methods such as, for example, MRI methods (Magnetic Resonance Imaging methods), it can be provided according to the invention that the flock material comprises a radiopaque additive such as, for example, barium sulphate, especially in the form of particles, or a metal or nitinol thread.

To achieve a biological, pharmaceutical and/or medical effect, it can be provided in an alternative or additional embodiment that the flock material comprises an active ingredient selected from the group comprising biological active ingredient, pharmaceutical active ingredient, medical active ingredient and mixtures thereof.

More particularly, the flock material can comprise an active ingredient selected from the group comprising antimicrobial, more particularly antibiotic, active ingredient, wound healing-promoting active ingredient, disinfecting active ingredient, anti-inflammatory active ingredient, blood coagulation-promoting active ingredient, growth factors, cell-differentiating factors, cell-adhesive factors, cell-recruiting factors, cell receptors, cell-binding factors, cytokines, peptides, structural proteins, extracellular proteins such as, for example, collagen, serum proteins such as, for example, albumin, polysaccharides such as, for example, hyaluronic acid, oligonucleotides, polynucleotides, DNA, RNA, salts thereof, stereoisomers, more particularly diastereomers, thereof and mixtures thereof.

For example, the flock material can comprise an active ingredient selected from the group comprising biguanides, polyhexamethylene biguanide (PHMB), triclosan, chlorhexidine, gentamicin, vitamins, copper, zinc, silver, gold, salts thereof, stereoisomers, more particularly diastereomers, thereof and mixtures thereof.

According to the invention, it can be further provided that the medical product is in the form of a mixture of different fibrous flock materials. In this case, the flock materials can differ from one another with regard to at least one parameter preferably selected from the group comprising fibre length, thickness or diameter, linear density, material and mixtures thereof. With regard to the parameters listed in this paragraph, full reference is made to the description so far.

The medical product is sprayable, more particularly aerosolizable.

In useful embodiments, the suspension referred to at the start in connection with the product according to the invention additionally comprises a biocompatible solution agent, dispersion agent or suspension agent, generally water or an aqueous mixture, and/or a biocompatible carrier substance such as, for example, fats, oils, glycerol or the like.

If the product according to the invention is present in the form of a suspension, it can be provided that the suspension has a flock material content of from 1% by weight to 20% by weight, more particularly from 3% by weight to 15% by weight, preferably from 5% by weight to 10% by weight, based on the total weight of the suspension.

In useful embodiments, the medical product is present in sterile and, in particular, off-the-shelf form. Sterilization of the product can, for example, be achieved using ethylene oxide and/or gamma irradiation.

It is further disclosed a discharge device containing the medical product described in the context of the first inventive aspect.

The discharge device is preferably designed as a spray device.

The discharge device can, in particular, be intended for discharging an aerosol. In this case, it may be useful for the discharge device to contain a suitable propellant in addition to the medical product. A suitable propellant can be selected from the group comprising perfluorinated ethers (for example Desfluran®), fluorinated hydrocarbons, more particularly fluorinated hydrocarbons of medium chain length (chain length having from two to five carbon atoms) such as, for example, tetrafluoroethane, hexafluoropropane, heptafluoropropane, decafluorobutane, octafluorocyclobutane, noble gases, nitrogen, oxygen, carbon dioxide, dinitrogen oxide and mixtures thereof.

With regard to further features and advantages of the discharge device, especially the medical product and/or a possible propellant, full reference is made to the description so far.

Lastly, in a second aspect, the invention provides a medical kit comprising the medical product described in the context of the first inventive aspect and also at least one further component selected from the group comprising a suture material, a stapler, a discharge device, more particularly a spray device, for the medical product and combinations thereof.

With regard to further features and advantages of the kit, in particular the medical product and/or the discharge device, full reference is made to the description so far.

Further features and advantages of the invention are revealed by the following description of preferred embodiments in the form of examples. Individual features can, in each case, be realized on their own or in combination with one another. The preferred embodiments serve solely to further elucidate the invention and to provide a better understanding of the invention, without restricting the invention thereto.

### Example section

### 1. Production of flock fibres composed of poly(ε-caprolactone-co-trimethylene carbonate)

An extrusion device was used to extrude fibres composed of poly(ε-caprolactone-co-trimethylene carbonate). Said fibres were subsequently trimmed to flock fibres of 100 µm in length using a precision cutting device. The produced flock fibres composed of poly(ε-caprolactone-co-trimethylene carbonate) were subsequently sterilized using ethylene oxide and lastly dried.

It was possible to use the flock fibres successfully in the coverage of wounds and as a haemostatic.

### 2. Production of particulate flock material composed of collagen (not part of the invention)

Starting from bovine skin, collagen-containing granules were produced and subsequently finely ground. This gave rise to a collagen-containing flock material having a particle size in the range of 10 µm to 80 µm, depending on the milling operation.

It was similarly possible to use the collagen-containing flock material successfully for wound coverage and stopping bleeding.

### 3. Treatment of a superficial wound

Using a scalpel, a 5 cm long incision was made on the right shank of a pig.

The resulting wound was cleaned. Thereafter, collagen-containing flock fibres of 100 µm in length were sprayed onto the wound until the wound was completely covered. Here, the flock fibres fixed in the wound by themselves. It was possible to easily remove excess flock fibres outside the wound.

The covered wound was subsequently further treated as part of a negative-pressure therapy, with the "porous" structure formed by the sprayed-on flock fibres allowing cell transfer and tissue transfer into the wound region and drying of the wound. At the same time as new skin formation, the flock fibres were resorbed, finalizing wound closure with endogenous skin.

## Claims

1. Medical product in the form of a fibrous flock material or of a suspension containing a fibrous flock material, wherein the fibrous flock material is present in the form of single flock fibers and the medical product is sprayable, **characterized in that** the flock material comprises a resorbable material or is formed from such a material.

2. Medical product according to claim 1, **characterized in that** the product is a wound covering agent, especially in the form of a spray bandage.

3. Medical product according to claim 1, **characterized in that** the product is a haemostatic.

4. Medical product according to any of the preceding claims, **characterized in that** the fibrous flock material is present in the form of monofilament flock fibres.

5. Medical product according to any of the preceding claims, **characterized in that** the fibrous flock material has a fibre length of from 10 µm to 5 mm, more particularly from 40 µm to 4 mm, preferably from 50 µm to 3 mm.

6. Medical product according to any of the preceding claims, **characterized in that** the fibrous flock material has a linear density of from 0.01 dtex to 100 dtex, more particularly from 0.1 dtex to 80 dtex, preferably from 0.3 dtex to 40 dtex.

7. Medical product according to any of the preceding claims, **characterized in that** the flock material comprises a material or is formed from a material which comprises functional groups selected from the group comprising carboxylate groups, sulphate groups, sulphonate groups, phosphate groups, amino groups and combinations thereof.

8. Medical product according to any of the preceding claims, **characterized in that** the flock material comprises a crosslinked material or is formed from such a material.

9. Medical product according to any of the preceding claims, **characterized in that** the flock material comprises a resorbable material or is formed from a resorbable material which is selected from the group comprising polyhydroxyalkanoates, proteins, extracellular proteins, serum proteins, polysaccharides, mucopolysaccharides, carboxyl group-bearing polysaccharides, amino group-bearing polysaccharides, aldehyde group-bearing polysaccharides, copolymers thereof, salts thereof, stereoisomers, more particularly diastereomers, thereof and mixtures, more particularly blends, thereof.

10. Medical product according to any of the preceding claims, **characterized in that** it is aerosolizable.

11. Medical kit comprising a medical product according to any of the preceding claims and at least one further component selected from the group comprising a suture material, a stapler, a discharge device, more particularly a spray device, for the medical product and combinations thereof.

## Patentansprüche

1. Medizinisches Produkt in Form eines faserförmigen Flockmaterials oder einer faserförmiges Flockmaterial enthaltenden Suspension, wobei das faserförmige Flockmaterial in Form von einzelnen Flockfasern vorliegt und das medizinische Produkt sprühbar ausgebildet ist, **dadurch gekennzeichnet, dass** das Flockmaterial ein resorbierbares Material aufweist oder aus einem solchen Material gebildet ist.

2. Medizinisches Produkt nach Anspruch 1, **dadurch gekennzeichnet, dass** es sich bei dem Produkt um ein Wundabdeckungsmittel, insbesondere in Form eines Sprühverbandes, handelt.

3. Medizinisches Produkt nach Anspruch 1, **dadurch gekennzeichnet, dass** es sich bei dem Produkt um ein Hämostyptikum handelt.

4. Medizinisches Produkt nach einem der vorhergehenden Ansprüche, **dadurch gekennzeichnet, dass** das faserförmige Flockmaterial in Form von monofilen Flockfasern vorliegt.

5. Medizinisches Produkt nach einem der vorhergehenden Ansprüche, **dadurch gekennzeichnet, dass** das faserförmige Flockmaterial eine Faserlänge von 10 µm bis 5 mm, insbesondere 40 µm bis 4 mm, bevorzugt 50 µm bis 3 mm, aufweist.

6. Medizinisches Produkt nach einem der vorhergehenden Ansprüche, **dadurch gekennzeichnet, dass** das faserförmige Flockmaterial einen Titer (längenbezogene Masse) von 0,01 dtex bis 100 dtex, insbesondere 0,1 dtex bis 80 dtex, bevorzugt 0,3 dtex bis 40 dtex, aufweist.

7. Medizinisches Produkt nach einem der vorhergehenden Ansprüche, **dadurch gekennzeichnet, dass** das Flockmaterial ein Material aufweist oder aus einem Material gebildet ist, welches funktionelle Gruppen aufweist, die ausgewählt sind aus der Gruppe umfassend Carboxylatgruppen, Sulfatgruppen, Sulfonatgruppen, Phosphatgruppen, Aminogruppen und Kombinationen davon.

8. Medizinisches Produkt nach einem der vorhergehenden Ansprüche, **dadurch gekennzeichnet, dass** das Flockmaterial ein vernetztes Material aufweist oder aus einem solchen Material gebildet ist.

9. Medizinisches Produkt nach einem der vorhergehenden Ansprüche, **dadurch gekennzeichnet, dass** das Flockmaterial ein resorbierbares Material aufweist oder aus einem resorbierbaren Material gebildet ist, welches ausgewählt ist aus der Gruppe umfassend Polyhydroxyalkanoate, Proteine, extrazelluläre Proteine, Serumproteine, Polysaccharide, Mucopolysaccharide, Carboxylgruppen tragende Polysaccharide, Aminogruppen tragende Polysaccharide, Aldehydgruppen tragende Polysaccharide, Copolymere davon, Salze davon, Stereoisomere, insbesondere Diastereomere, davon und Mischungen, insbesondere Blends, davon.

10. Medizinisches Produkt nach einem der vorhergehenden Ansprüche, **dadurch gekennzeichnet, dass** es aerosolierbar ist.

11. Medizinisches Kit, umfassend ein medizinisches Produkt nach einem der vorhergehenden Ansprüche und wenigstens eine weitere Komponente, welche ausgewählt ist aus der Gruppe umfassend ein Nahtmaterial, ein Stapler, eine Austragvorrichtung, insbesondere eine Sprühvorrichtung, für das medizinische Produkt und Kombinationen davon.

## Revendications

1. Produit médical sous la forme d'une matière floculée fibreuse ou d'une suspension contenant une matière floculée fibreuse, où la matière floculée fibreuse est présente sous la forme de fibres floculées uniques et le produit médical est pulvérisable, **caractérisé en ce que** la matière floculée comprend une matière résorbable ou est formée d'une telle matière.

2. Produit médical selon la revendication 1, **caractérisé en ce que** le produit est un agent de couverture de plaies, spécialement sous la forme d'un bandage pulvérisable.

3. Produit médical selon la revendication 1, **caractérisé en ce que** le produit est un hémostatique.

4. Produit médical selon l'une quelconque des revendications précédentes, **caractérisé en ce que** la matière floculée fibreuse est présente sous la forme de fibres floculées monofilamentaires.

5. Produit médical selon l'une quelconque des revendications précédentes, **caractérisé en ce que** la matière floculée fibreuse présente une longueur de fibres comprise entre 10 µm et 5 mm, plus particulièrement entre 40 µm et 4 mm, préférentiellement entre 50 µm et 3 mm.

6. Produit médical selon l'une quelconque des revendications précédentes, **caractérisé en ce que** la matière floculée fibreuse présente une densité linéaire comprise entre 0,01 dtex et 100 dtex, plus particulièrement entre 0,1 dtex et 80 dtex, préférentiellement entre 0,3 dtex et 40 dtex.

7. Produit médical selon l'une quelconque des revendications précédentes, **caractérisé en ce que** la matière floculée comprend une matière ou est formée d'une matière qui comprend des groupements fonctionnels choisis dans le groupe comprenant les groupements carboxylate, les groupements sulfate, les groupements sulfonate, les groupements phosphate, les groupements amino et leurs combinaisons.

8. Produit médical selon l'une quelconque des revendications précédentes, **caractérisé en ce que** la matière floculée comprend une matière réticulée ou est formée d'une telle matière.

9. Produit médical selon l'une quelconque des revendications précédentes, **caractérisé en ce que** la matière floculée comprend une matière résorbable ou est formée d'une matière résorbable qui est choisie dans le groupe comprenant polyhydroxyalcanoates, protéines, protéines extracellulaires, protéines sériques, polysaccharides, mucopolysaccharides, polysaccharides portant des groupements carboxyle, polysaccharides portant des groupements amino, polysaccharides portant des groupements aldéhyde, les copolymères de ceux-ci, les sels de ceux-ci, les stéréoisomères, plus particulièrement les diastéréoisomères, de ceux-ci et les mélanges, plus particulièrement les formulations, de ceux-ci.

10. Produit médical selon l'une quelconque des revendications précédentes, **caractérisé en ce qu'**il peut être aérosolisé.

11. Kit médical comprenant un produit médical selon l'une quelconque des revendications précédentes et au moins un composant supplémentaire choisi dans le groupe comprenant une matière de suture, une pince à suture, un dispositif de libération, et particulièrement un dispositif de pulvérisation, pour le produit médical et leurs combinaisons.
